# EUROPEAN PATENT SPECIFICATION

(11) **EP 0 518 319 B1**
(45) Date of publication and mention of the grant of the patent: **24.03.1999**
(21) Application number: 92109828.1
(22) Date of filing: 11.06.1992
(51) Int. Cl.: G01N 33/58, G01N 33/543, G01N 33/94

(54) **Liposome immunoassays for detection of antigens, antibodies and haptens**
Liposome-Immunoassay zum Nachweis von Antigenen, Antikörpern und Haptenen
Essai immunologique à base de liposomes pour la détection des antigènes, des anticorps et des haptènes

(30) Priority: 12.06.1991 IL 98473
(43) Date of publication of application: 16.12.1992
(73) Proprietor: YEDA RESEARCH AND DEVELOPMENT COMPANY LIMITED, Rehovot 76100 (IL)
(72) Inventor: Gitler, Carlos, Weizmann Institute of Science, Rehovot (IL)
(74) Representative: VOSSIUS & PARTNER

(56) References cited:
- EP-A- 0 160 266
- EP-A- 0 254 117
- EP-A- 0 301 333
- FR-A- 2 538 907
- US-A- 4 176 174
- US-A- 4 874 710
- JOURNAL OF IMMUNOLOGICAL METHODS, vol. 70, no. 2, 25 May 1984, New York, NY (US); J.W. FREYTAG et al., pp. 133-140/
- DATABASE WPIL, Week 9142, Derwent Publications Ltd., London (GB); AN 91-307696/

## Description

The present invention relates to an improved liposome immunoassay.

Liposome immunoassays for the detection of antigens, antibodies and haptens are sensitive and fast and constitute a very important tool for immunologic research and accurate diagnosis in clinical analyses (for a review, see: Monroe, D., "Novel Liposome Immunoassays for Detecting Antigens, Antibodies and Haptens" (1990) J.Liposome Res. **1(3)**: 339-377).

Liposome immunoassay is based on immune lysis of the liposome vesicles, thus releasing a marker occluded within the inner volume of the liposome, the amount of reagent released serving as a quantitative indicator of the reaction taking place. A variety of markers are used in these assays, such as sugars, specific ions, electron spin molecules and, most often, enzymes and dyes. The liposome lysis is achieved either by the addition of complement, which binds to immune complexes formed on the liposomal surface and initiates the complement cascade leading to liposome lysis, or by a cytolytic agent, such as marine worm protein Cerebratulus lacteus toxin A-III, Central Asia cobra venom and bee venom melittin, covalently linked to an antibody or antigen.
European Patent Application EP 301 333 refers to such an assay for detection of an analyte in a fluid sample by an antibody involving complement-mediated lysis of marker-encapsulating liposomes coupled with said antibody. Complement is activated for lysis of the liposomes by a second antibody directed to the analyte. Lysis takes place upon formation of the liposome-antibody-antigen-second antibody complex. Liposomes used for the assay are not embedded into a matrix.
US Patent No. 4,874,710 discloses a liposome assay comparable to liposome immunoassays, however, using enzymes as the preferred lysis agents. This document discloses a competitive assay for detecting an analyte in a sample using sacs, such as liposomes, which include a detectable marker. Thereby, a conjugate comprised of a ligand and a sac lysing agent binds to the analyte or directly to a binder or to the binder through the analyte, whereby the amount of conjugate bound to the binder depends upon the amount of analyte in the sample. Conjugate not bound to the binder (directly or indirectly) comes into contact with the sacs thereby lysing them which results in the release of the marker. In order to avoid the binder-conjugate complex having free access to the sacs which are free to move in the assay medium or are bound on a solid support, however not within a solid support, the binder is supported on an appropriate solid medium.

Although liposome immunoassays in general have many advantages and applications in comparison to traditional immunoassays and offer a specific and sensitive method for detecting minute quantities of analytes, there are still difficulties to be solved in order to make this technique more attractive and more reliable for diagnostic purposes. Among these problems are the liposome stability and capacity to retain the marker occluded in its inner volume, without any or with only a minimal leakage to the medium, and a higher sensitivity of the assay through improvement of the signal to noise ratio of the assay.

It has now been found according to the present invention that liposomes entrapped or enmeshed in a polymer network are very stable and suitable for liposome immunoassay, increasing many fold the sensitivity of the immunoassay.

Entrapped liposomes have been proposed for slow release of drugs. European Patent Application EP 160 266 describes a liposome composition comprising a three-dimensional crosslinked matrix physically trapping a liposome enclosing a material to be released into a living body at a slow rate. The material to be administered into a living body may be a medicine, particularly insulin, heparin, urokinase, ubidecarenone and cytosine arabinoside, or a marker, plasmid, DNA or RNA which prove effective when administered into the living body. The three-dimensional network structure physically entrapping the liposome containing the medical material is described as acting not only as a buffer, to prevent the liposome from directly contacting a body fluid, to be broken in a short time, but also as a support capable of securely holding the liposome in the matrix, thus enabling the material held in the liposome film to be released into the living body at a satisfactorily slow rate.

In contrast to the teaching of the prior art, where the material enclosed within the entrapped liposomes is released at a slow rate, the marker occluded within the enmeshed liposomes of the present invention is released immediately, at a very fast rate, thus improving the signal to noise ratio of the assay.

According to the present invention, a liposome immunoassay to detect an antibody, an antigen or a hapten in a sample is provided wherein the assay is carried out with liposomes enmeshed in a polymer network, said enmeshed liposomes having an occluded marker within their inner volume. The liposomes undergo complement- or cytolytic agent-mediated immune lysis, the marker is released into the supernatant and qualitatively estimated or quantitatively measured. The amount of marker indicates the concentration of the antibody, antigen or hapten in the sample.

As used herein the term "analyte" comprises an antibody, an antigen or a hapten, and the term "hapten" means a molecule which is not antigenic by itself, but becomes antigenic when attached to another large molecule.

The liposomes used in the present invention may be composed of any suitable natural or synthetic phospholipid, such as phosphatidylcholine, phosphatidylethanolamine, phosphatidylinositol, phosphatidylglycerol, phosphatidylserine or sphingomyelin which are derived from egg yolk, soybean and animal tissues, egg yolk lecithin and soybean lecithin which are a mixture of the above-noted phospholipids, or synthetic lecithin such as dipalmitoyl lecithin and distearoyl lecithin. They may also contain sterols, such as cholesterol, an anti-oxidant, such as α-tocopherol, more or less ionic surface active substances such as dicetyl phosphate, stearylamine or phosphatidic acid, and/or other materials of a hydrophobic nature. Preferably, the liposomes of the invention comprise soybean phospholipids, cholesterol and α-tocopherol, in a ratio varying according to the composition of the phospholipid material, such as, for example 100:10:1 for soybean phospholipids, cholesterol and α-tocopherol, respectively.

The polar groups of phospholipids, such as phosphatidylethanolamine, contain amino groups that can be used to attach hapten moieties to the liposomal outer surface, e.g., trinitrobenzene sulfonic acid, resulting in end products, e.g., N-trinitrophenylphosphatidylethanol amine, that will react with antibodies that recognize the hapten.

The marker occluded in the liposomes may be any marker used in liposome immunoassays. The choice of the marker is important in order to achieve maximal stability, sensitivity and reproducibility. Ions, such as F⁻, sugar (glucose) and electron spin molecules may be used as liposome-entrapped labels, but the preferred markers are fluorescent dyes and enzymes, that usually enhance the assay sensitivity, improve reproducibility and can be easily monitored spectrophotometrically, spectrofluorimetrically or potentiometrically. Water-soluble fluorescent dyes and enzymes are preferably used in the invention since their concentration in the supernatant can be easily determined in situ without the need for a separation step from the liposomes. Examples of dyes that can be used in the invention are the fluorochromes calcein, carboxyfluorescein and sulforhodamine B. They are conveniently measured by flow cytometry, spectrophotometry, spectrofluorometry, filter fluorometry, and fluorescent microscopy. Examples of enzymes are horseradish peroxidase, alkaline phosphatase, β-galactosidase, glucose oxidase, etc. The released enzyme marker is immediately available to act upon a larger amount of specific substrate, that will in turn chemically change into an end product that can be measured. For example, alkaline phosphatase occluded within the liposomes is released upon lysis and acts upon colorless p-nitrophenyl phosphate substrate to produce colored p-nitrophenol that can be detected spectrophotometrically at 410 nm.

The polymer network may consist of any suitable gel that does not damage the liposome, nor interferes with the assay. High molecular weight materials such as cross-linked polyacrylamide, polymethacrylic acid, polyethylene oxide and similar materials may be used. Polymerization of a monomer, such as acrylamide, in the presence of liposomes and of a cross-linking agent, such as N,N'-methylene-bis-acrylamide, leads to formation of the polymer in the aqueous space surrounding the liposomes, resulting in a three-dimensional polymer network entrapping or enmeshing the liposomes without lysing them. The size of the meshwork will depend both on the amount of acrylamide and of the cross-linking agent.

The polymer-enmeshed liposomes of the invention containing an occluded marker can be obtained by several known procedures. Multilamellar liposomes are formed by allowing phospholipids and other lipids spread as thin, dry multilamellar sheets to swell by incorporating water between the phospholipid bilayers or lamellae. When swelling is performed in a solution that contains an adequate solute, e.g. a marker, subsequent short exposure to ultrasonic vibration results in breaking down of the lamellar sheets to form multilamellar closed vesicles containing the solute. Dialysis in a sack against a solution that is isotonic with the liposome, but contains no solute, or passage through an adequate chromatographic material, will cause removal of solutes not entrapped within the liposome inner volume. Thus only vesicles containing the solute, such as a marker, entrapped in their inner volume, will be obtained. Unilamellar liposomes can be formed by removal of a detergent that solubilizes the phospholipids or by addition of an aqueous solution to ether, sonication and subsequent removal of the ether.

According to the invention, purified liposomes in solution are mixed with a mixture of the monomer and the cross-linking agent and subjected to polymerization, whereby enmeshed liposomes containing an occluded marker are obtained. For example, liposomes containing calcein or horseradish peroxidase, are mixed with a mixture of acrylamide and methylene-bis-acrylamide, in a final concentration of 4-5%:0.13%, respectively, and the mixture is subjected to polymerization, e.g., with ammonium persulfate-N, N, N', N'-tetramethylethylenediamine (Temed) in usual ratios, as initiator. Once polymerization is finished and a gel material is obtained, it may be subjected to fragmentation by any suitable method, for example, passing the gel through needles, by forcing with a syringe. Needles of 16 to 27½ Luer are suitable. Following the final passage, the gel-enmeshed liposome suspensions are washed by sedimentation with an isotonic solution, such as Hepes-K⁺ buffer (50 mM Hepes, 100 mM K₂SO₄, pH 7.4), the supernatant is discarded and the liposomes are then used in the assay. In an alternative procedure, microspheres with enmeshed liposomes may be obtained directly from the polymerization step.

A hapten, antigen or antibody may be attached to the liposomal surface by any means that allows adequate liposome surface presentation of the molecule: chemically, by adsorption, via electrostatic force or any other suitable means. When chemically bound, the molecule may be involved in immunological reaction on the surface of the polymer-enmeshed liposomes.

In one embodiment, the polymer-enmeshed liposomes of the invention with a hapten attached to the outer surface, are prepared by a method which comprises the following steps:
i) mixture of suitable phospholipids, a sterol and an anti-oxidant in solution and evaporation of the solvent;
ii) addition of the marker solution and sonication, thus obtaining liposomes with occluded marker;
iii) polymerization of a monomer and a cross-linking agent in the presence of the liposomes with occluded marker of step (ii), and fragmentation of the polymer-enmeshed liposomes, such as to increase the surface area;
iv) reaction of the polymer-enmeshed liposomes of step (iii) with a hapten in isotonic buffer solution; and
v) sedimentation and washing, thus recovering the desired polymer-enmeshed liposomes.

In an alternative procedure, the liposomes containing an occluded marker of step (ii) are first reacted with the hapten and then subjected to polymerization. When no hapten is needed, this step is ommitted. Similarly, the polymer-enmeshed liposomes of step (iii) may react in step (iv) with an antigen or antibody.

According to the invention, a direct liposome immunoassay of an antibody, antigen or hapten is provided, which comprises the following steps:
a) addition of a sample containing the antibody, antigen or hapten to polymer-enmeshed liposomes having a marker occluded within their inner volume;
b) incubation of the suspension with an agent to cause liposome lysis, with subsequent release of the marker into the supernatant;
c) sedimentation of the suspension under normal gravity; and
d) measurement of the marker released from the lysed liposomes in the supernatant.

The agent causing the liposome lysis may be complement or a cytolytic agent.

In complement-mediated liposome lysis, the polymer enmeshing the liposomes will have a low cross-linking degree, for example, ≤5% for acrylamide gel, thus enabling antibody molecules from the sample to penetrate into the gel matrix and to reach the liposome surface. The complement may be already present in the sample, for example in fresh immune serum, or may be added externally from a suitable source, such as commercially available guinea pig complement.

In the cytolytic agent - mediated liposome lysis, the polymer enmeshing the liposomes will have a high cross-linking degree, for example, above 10% for acrylamide gel, thus preventing diffusion of high molecular weight antibody molecules, but allowing penetration of low molecular weight cytolytic agents. Any cytolytic agent may be used, such as bee venom melittin, synthetic peptides having melittin-like cytolytic activity, e.g., the peptide called CH-1, a melittin homolog of 22 amino acids, described hereinafter, and phospholipases, for example those derived from Central Asia cobra venom and marine worm **Cerebratulus lacteus** toxin A-III.

As described above, when a cytolytic agent is used, the polymer meshwork enmeshing the liposomes will be so constructed that it acts as a selective barrier for the diffusion of molecules of various molecular weights to the liposome surface. Thus, the polymer meshwork will allow the penetration of small molecules, such as of a [cytolytic agent-hapten] conjugate, but will prevent diffusion of large molecules, such as of an antibody or an [antibody-cytolytic agent-hapten] complex. A 10-12% cross-linked polyacrylamide gel will, for example, fulfill these requirements.

For assay of antibodies, e.g., against microorganisms or AIDS, in the presence of complement, serum is incubated with polymer-enmeshed liposomes having a hapten comprising the epitope for the antibody, attached to the outer liposomal surface. If the serum is not fresh, complement is added to the sample. The release of the occluded marker is used to measure liposome lysis.

For quantitative assay of antigens or small molecular weight analytes present in a serum or other sample, the procedure involves two steps. First, an antibody specific for the analyte is added in excess of the free analyte to be measured. Then, the fraction of the antibody that was not neutralized is determined by lysis of hapten-containing liposomes in the presence of complement. The hapten attached to the liposomes, in this case, will have the same chemical features as the analyte.

A cytolysis-mediated liposome immunoassay of an antibody in a sample comprises :
(a) incubation of the sample with known concentrations of a conjugate of a cytolytic agent and a hapten recognized by the antibody, in excess to the antibody to be measured, so as to form an [antibody-cytolytic agent-hapten] complex;
(b) addition of the sample containing the complex and free [cytolytic agent-hapten] conjugate to polymer-enmeshed liposomes having an occluded marker, such that only the conjugate will penetrate into and reach the liposome surface causing liposome lysis and subsequent release of the marker into the supernatant;
c) sedimentation of the suspension under normal gravity, and
d) measurement of the marker released from the lysed liposomes in the supernatant.

The direct immunoassay of an antigen or a hapten in a sample, in the presence of cytolytic agent, will comprise the following steps:
(a) incubation of a mixture of known amounts of an antibody against the antigen or hapten with a conjugate of a cytolytic agent with a hapten recognized by the antibody, resulting in neutralization and formation of a 1:1 [antibody-cytolytic agent-hapten] complex, with no free antibody or [cytolytic agent-hapten] conjugate;
(b) addition of mixture obtained in (a) to the sample containing the antigen or hapten to be measured, so that the antigen or hapten will compete for the antibody in the complex and will release [cytolytic agent-hapten] conjugate;
(c) addition of highly crosslinked polymer-enmeshed liposomes having a marker occluded within their inner volume, to mixture (b) and incubation of the suspension, such as to cause liposome lysis by the free [cytolytic agent-hapten] conjugate, and release of the marker into the supernatant;
(d) sedimentation of the suspension; and
(e) measurement of the marker released from the lysed liposomes in the supernatant.

In another embodiment of the invention, the polyacrylamide-enmeshed liposomes are kept solid-like and are part of the wall of a multiwell microtiter plate, forming a receptacle for the sample. Then, simple addition of immune serum results in the release of the entrapped marker, e.g. calcein, giving a fluorescent halo around the polyacrylamide wall.

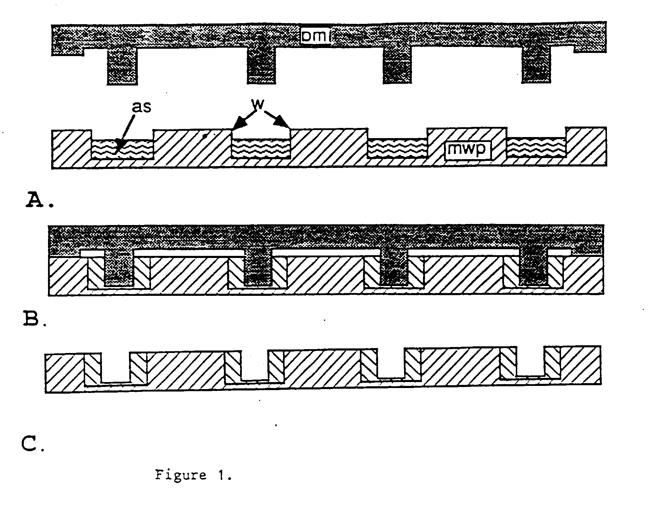

Figure 1 illustrates the methodology to create a solid support where the acrylamide-enmeshed liposomes are part of a multiwell microtiter plate. Formation of a transparent polyacrylamide layer in the wells of a multiwell plate is carried out as follows : (A). a 10% acrylamide-methylene-bis-acrylamide solution (as) is placed in the wells (w) of a plastic multiwell plate (mwp). A plastic mold (pm) is made such that it can fit into the wells; (B) polymerization of the acrylamide (as) with the mold (pm) in place; (C) withdrawal of the mold resulting in formation of transparent polyacrylamide layers in the wells (diagonal lines, top left to bottom right), ready for the introduction of the acrylamide-enmeshed liposomes.

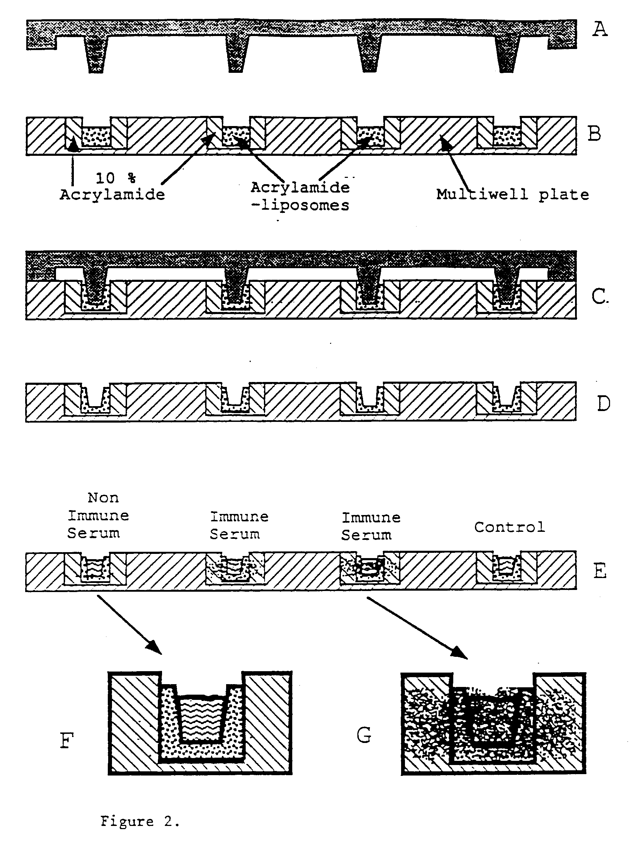

Figure 2 illustrates formation of the acrylamide-enmeshed liposome wells and the appearance of the wells on reacting with immune and non-immune sera. To each well of the multiwell microtiter plate containing the polyacrylamide layer of Figure 1C, here represented by B, is added a mixture of acrylamide-methylene-bis-acrylamide and liposomes with occluded calcein having a hapten group (N-trinitrophenyl phosphatidylethanolamine) in the surface (black square dots). Immediately after addition of the polymerization initiator (Temed and ammonium persulfate) to each well, the mold A is introduced to give an ensemble as shown in C. Withdrawal of mold A after polymerization, gives a multiwell microtiter plate with the geometry shown in D. Wells are created which are lined by polyacrylamide-enmeshed liposomes with a specific hapten on their surface and with self-quenched calcein within their inner volume (black square dots) over the original transparent layer. To these wells, fresh sera or control (PBS) is added directly as shown in E. After incubation for 15 to 30 min, the immune sera containing an antibody to be determined (anti-trinitrophenyl antibodies) and complement, diffuse into the acrylamide gel and result in the specific lysis of the liposomes and release of the calcein (G). The black dots represent the calcein fluorescence that is very high, because the calcein is released to the external medium of the liposomes where it is diluted and loses its self-quenching. When non-immune serum (not containing antibodies to the hapten present at the liposome surface), is added to the well (F), almost no lysis occurs.

The polymer-enmeshed liposome immunoassay of the invention has several advantages in comparison with the known immunoassay performed with free non-enmeshed liposomes:
1. The liposomes enmeshed in the polymer network, preferably polyacrylamide, have an enhanced shelf-life. The stability and capacity to retain the occluded fluorogenic and enzymatic markers is enhanced by at least 40% over a 3 month period. That is, the loss of solutes is some 40% less in the polyacrylamide-enmeshed liposomes in comparison to the equivalent free liposomes.
2. The degree of cross-linking of the polymer, e.g. acrylamide-methylene-bis-acrylamide gel, determines the size of the molecules that can penetrate into the gel matrix to interact with the liposomes. By increasing the cross-linking degree of the polymer, a selective barrier can be designed.
3. The polymer-enmeshed liposomes are stabilized by the polymer mesh. Furthermore, any released fluorophore can be readily removed by the sedimentation of the enmeshed liposomes under normal gravity, by decanting the supernatant with the free fluorophore, thus achieving a dramatically increased signal to noise ratio.
4. The use of polyacrylamide-enmeshed liposomes increases the sensitivity of the immunoassay. The enhancement in the signal obtained on immune lysis of the polyacrylamide-enmeshed liposomes is about 250 when compared with the same sample incubated in the presence of non-immune sera. The free liposomes give only an enhancement of about 9 fold. The reasons for this difference are that:
   a) Due to higher specific gravity, removal of the released free fluorescent molecules is very difficult with free liposomes, but very effective with the polymer-enmeshed liposome gel fragments, because they sediment under normal gravity on standing for a few minutes (1-2 minutes), and do not require any, or only gentle, centrifugation. This is very suitable for private clinics.
   b) The liposomes that did not undergo immune lysis remain in the light pathway when free liposomes are used, since they cannot be removed by any simple procedure. On the other hand, the polyacrylamide-enmeshed liposome gel fragments can be removed readily by sedimentation below the light path and thus do not contribute to the measured signal, i.e.fluorescence signal.
5. Derivatization of the liposomes to attach haptens to their outer surface can be performed while they are enmeshed. This results in saving of great effort and increases significantly the final yield, because no passage through columns is required, a procedure that leads to losses of some 15 to 20% of the liposomes during each column step. In comparison, the gel fragments containing the enmeshed liposomes can be sedimented at each step and washed with quantitative recoveries.
6. Polyacrylamide-enmeshed liposomes can be kept solid and allow geometric design of wells that permit multiwell plates to be made where qualitative analysis can be readily peformed. The layer formed by polyacrylamide-enmeshed liposomes in the multiwell plate may be made very thin so that reaction with the sample will occur over a large surface and diffusion of the released marker into the transparent polyacrylamide layer will be very fast.

The invention will now be illustrated by the following non-limiting examples.

### Example 1. Preparation of polyacrylamide-enmeshed liposomes with occluded calcein

**1.1** 0.2 ml of a benzene solution containing 40 mg (0.05 mmoles) of Sigma soybean phospholipids type II were mixed well with 10 µl of a benzene/methanol 9:1 solution containing 1.93 mg of cholesterol and 10 µl of a benzene solution containing 2.5 mg of α-tocopherol, in a heavy-walled culture tube. The solvent was evaporated under nitrogen, thus obtaining a lipid film on the bottom of the tube.
**1.2** Calcein in acid form was neutralized with KOH by suspending 44 mg of acid calcein in 600 µl water, 50 µl 1 M Hepes buffer, pH 7.4, adding 1 M KOH until the pH is 7.4, and adjusting the volume to 1.0 ml. Final concentrations were: calcein, 70 mM; Hepes, 50 mM. The osmolarity of the solution was about 310-320 mOsm. 1.0 ml of this calcein solution was added to the lipid film of step (1.1), glass beads were added thereto and the whole was agitated in strong vortex for 10 min. The mixture was then sonicated in a bath sonicator for 1 min and freeze-thawed twice slowly using dry ice. It was then placed in a dialysis tube open at one end and dialyzed against Hepes-K+ buffer (50 mM Hepes, 100 mM K₂SO₄, pH 7.4).
**1.3** In order to obtain a 5% gel, to 165 µl of an aqueous solution of 30% acrylamide-0.8% methylene-bis-acrylamide in a glass tube were added 50 µl of 1 M Hepes, pH 7.4, 100 µl of 1 M K₂SO₄ and 585 µl of water, and mixed. Then 100 µl of the above calcein-containing liposomes were added to the mixture and the polymerization was initiated with 5 µl of 10% ammonium persulfate-1 µl of N, N, N', N'-tetramethyl-ethylenediamine (Temed) 6.6 M. Once the gel had polymerized, it was removed from the tube, suspended in an equal volume of Hepes-K+ buffer and passed through needles of 16 to 27½ Luer, by forcing the suspension through the needle with a syringe. Following the final passage, the gel suspension was washed by sedimenting it from an isotonic solution of Hepes-K+ buffer. The gel-enmeshed liposome suspension is ready for hapten attachment or for immunoassay procedure in the presence of a cytolytic agent, when no hapten attachment is needed.

### Example 2. Preparation of hapten-attached polyacrylamide-enmeshed liposomes with occluded calcein

For the detection of anti-trinitrobenzene and/or anti-dinitrobenzene antibodies in immune sera, polyacrylamide-enmeshed liposomes containing N-trinitrophenyl-phosphatidylethanolamine(TNP-PE) may be prepared by reacting trinitrobenzenesulfonic acid either with non-enmeshed (procedure 2.1) or with polyacrylamide-enmeshed liposomes (procedure 2.2).
**2.1** Liposomes with occluded calcein prepared as in Example 1.2 were reacted with trinitrobenzenesulfonic acid (TNB) (20 mM final) in isotonic phosphate buffer, pH 8.5. The reaction was followed by the change of absorbance at 420 nm. When all the accessible lipids had reacted (two hours), the lipids were dialyzed against Hepes-K+ buffer. The liposomes were then enmeshed in cross-linked polyacrylamide as in Example 1.3.
**2.2** In another procedure, 5 ml of polyacrylamide-liposome gel suspension of Example 1.3, after fragmentation, were placed in isotonic phosphate buffer, pH 8.5, and trinitrobenzenesulfonic acid (20 mM final) was added thereto. The suspension was reacted as in 2.1 above for the non-enmeshed liposomes and then the free trinitrobenzenesulfonic acid was removed by sedimentation of the gel and washing with Hepes-K+ buffer until all the reagent was removed.

### Example 3. Direct immunoassay of anti-trinitrobenzene antibodies in immune serum in the presence of complement

To 50 µl of the calcein-containing polyacrylamide-enmeshed liposomes containing the N-trinitrophenyl phosphatidylethanolamine of Example 2, were added 50 µl of non-immune or of fresh undiluted immune serum or various dilutions to test the specificity of the lysis response due to formation of antigen-antibody complexes on the liposome surface, initiating the lytic effect of the complement (human non-immune serum was used as a source of complement). The suspension was gently incubated at 37°C and, after the appropriate time (30 min), 100 µl of Hepes-K+ buffer were added, the suspension was sedimented and the calcein in the supernatant was measured by its fluorescence at 485 nm excitation, 515 nm emission. The total calcein present in the liposomes was determined by treating an equivalent fraction of the liposome gel with Triton® X-100, 0.1% final, instead of the antibody. The results are shown in Table 1.

**Table 1**

| **Complement-mediated liposome direct immunoassay of immune serum containing antibodies against the trinitrophenyl moiety with polyacrylamide-enmeshed liposomes containing occluded calcein** | | | | | |
|---|---|---|---|---|---|
| Addition Serum | Serum dilution | Fluorescence | Standard deviation | Percent release | Percent baseline |
| None | - | 132 | 30 | 0 | 100 |
| Non-immune | - | 200 | 18 | 1 | 152 |
| Immune | 1:50 | 18340 | 230 | 63 | 13894 |
| Immune | 1:500 | 14754 | 231 | 50 | 11177 |
| Immune | 1:1000 | 6498 | 95 | 22 | 4923 |
| Immune | 1:5000 | 2365 | 121 | 8 | 1792 |
| 0.1% Triton® X-100 | | 29344 | 346 | 100 | 22230 |

For comparison, the assay was repeated with similar liposomes, but not enmeshed within a polymer network. The results are shown in Table 2.

**Table 2**

| **Complement-mediated liposome direct immunoassay of immune serum containing antibodies against the trinitrophenyl moiety with non-enmeshed liposomes containing occluded calcein** | | | | | |
|---|---|---|---|---|---|
| Addition Serum | Serum dilution | Fluorescence | Standard deviation | Percent release | Percent baseline |
| None | - | 3206 | 308 | 11 | 100 |
| Non-immune | - | 3562 | 180 | 12 | 111 |
| Immune | 1:50 | 19240 | 130 | 67 | 600 |
| Immune | 1:500 | 15428 | 161 | 53 | 481 |
| Immune | 1:1000 | 7028 | 90 | 24 | 219 |
| Immune | 1:5000 | 2436 | 111 | 8 | 75 |
| 0.1% Triton® X-100 | | 28773 | 456 | 98 | 897 |

The results with polymer enmeshed- and non-enmeshed liposomes show that the immunoassay using free (non-enmeshed) liposomes gives an enhancement between the non-lysed (addition=none - 11% release) and that completely lysed by Triton® X-100 (98% release) of 9 fold (Table 2), while the same immunoassay with the polyacrylamide-enmeshed liposomes gives an enhancement of about 250 (Table 1). This is due to the fact that the non-lysed polyacrylamide-enmeshed liposomes are spun down and only the liberated calcein in the supernatant is seen in the light path, when the measurement of fluorescence is performed, thus enhancing significantly the sensitivity of the assay. The 9 fold enhancement obtained here with non-enmeshed liposomes is very high and due to the fact that the liposomes were always kept within a dialysis sack. The usual enhancement factor is about 5 to 6. The liposome lysis is proportional to the antibody concentration in the serum; dilution of the serum with lower antibody concentration leads to a fall in the lysis and less fluorescence is measured.

### Example 4. Preparation of polyacrylamide-enmeshed liposomes containing occluded horseradish peroxidase

The liposomes were prepared by reverse-phase evaporation according to Szoka Jr., F., and Papahadjopoulos, D., (1978) Proc.Nat.Acad.Sci. USA **75**: 4194-4198. In this procedure, aqueous buffer is introduced into a solution of lipids in ether, an emulsion is created by sonication and the ether is evaporated to form large unilamellar liposomes (LUVs). The external solutes are removed by dialysis and the liposomes are then enmeshed in polyacrylamide gels.
**4.1** 0.2 ml of a benzene solution containing 24.8 mg of Sigma soybean phospholipids type II, 10 µl of benzene solution containing 0.595 mg cholesterol and 10 µl of a benzene solution containing 2.5 mg α-tocopherol were placed at the bottom of a 50 ml round-bottomed flask and mixed well. The solvent was evaporated under nitrogen. 5 ml of diethyl ether were added and the lipids were dissolved by gentle swirling of the flask.
**4.2** 1.5 ml of an aqueous medium containing horseradish peroxidase (2.0 mg of horseradish peroxidase Sigma P6782 (1200 units/mg) dissolved in 1.5 ml of Hepes-K⁺ buffer) were added to the liposome ether solution of step 4.1 above and the solution was sonicated in the bath sonicator for 2 to 3 min, until a transparent or slightly opalescent suspension was formed. The emulsion should not separate during standing for at least 30 min. The ether was evaporated until no odor of ether remained in the Buchii evaporator with water at 25°C, and the liposomes were dialyzed through a column of Sepharose 4B equilibrated in Hepes-K⁺ buffer to remove the free peroxidase.
**4.3** For preparation of a 5% gel, the procedure of Example 1.3 was repeated with the horseradish peroxidase-containing liposomes of 4.2 above.

### Example 5. Preparation of hapten-attached polyacrylamide-enmeshed liposomes with occluded horseradish peroxidase

For the detection of anti-fluorescein isothiocyanate (FITC) antibodies in immune sera, polyacrylamide-enmeshed liposomes having as hapten attached to the outer liposomal surface moieties of 1-(succinimidyl)propionyl-3-dithio-2-pyridine(SPDP) were prepared as follows:

Polyacrylamide-enmeshed liposomes containing occluded horseradish peroxidase of Example 4 were reacted with a 1.2 molar excess (with respect to the amino groups present at the liposome surface) of 1-(succinimidyl)propionyl-3-dithio-2-pyridine (SPDP, Pharmacia). The reaction was performed in Hepes-K⁺buffer, pH 8.5. The excess SPDP was removed by washing in Hepes-K⁺ buffer, pH 7.4. The antigen linked to a protein carrier, FITC-soybean trypsin inhibitor, was also reacted with SPDP under the same conditions and immediately before use, was treated with 0.1 mM of dithiothreitol, and passed through a Sephadex G-25 column to remove the excess dithiothreitol. The FITC-soybean trypsin inhibitor antigen containing a free thiol group derived from the SDPD was mixed with the polyacrylamide-enmeshed liposomes and the reaction was followed by the liberation of 2-thiopyridone. When the reaction was complete (30 min), the liposomes entrapped in the acrylamide gel were washed with Hepes-K⁺ buffer, pH 7.4.

### Example 6 Direct antibody immunoassay based on the horseradish peroxidase reaction in the presence of complement

The polyacrylamide-enmeshed liposomes (20 µl) containing the surface-bound FITC-soybean trypsin inhibitor of Example 5 were placed at the bottom of three conical tubes. To the first were added 20 µl of normal serum, to the second 20 µl of the test serum containing anti-FITC antibodies, and to the third 20 µl of the test serum containing anti-FITC antibodies and, in addition, an excess of the free FITC-soybean trypsin inhibitor. The tubes were left at room temperature for 15 min and then 50 µl of 50 mM Hepes,100 mM K₂SO₄, pH 6.0 containing 0.025 mM 2,2'-azino-bis(3-ethylbenzthiazoline-6-sulfonic acid) as a substrate for the peroxidase, and 0.25 mM hydrogen peroxide, were added and the components were incubated for 15 min at room temperature. The color developed was read at 412 nm. The results are given in Table 3.

**Table 3**

| **Complement-mediated liposomes direct immunoassay of immune serum containing anti-FITC antibodies with polyacrylamide-enmeshed liposomes containing occluded horseradish peroxidase** | | | |
|---|---|---|---|
| Addition Serum | Serum dilution | Peroxidase OD at 412 n m | Percent release |
| None | - | 0.014±0.003 | 0.4 |
| Non-Immune Serum | - | 0.011±0.003 | 0.3 |
| Immune Serum | 1/50 | 1.80±0.06 | 48 |
| | 1/500 | 1.42±0.05 | 38 |
| | 1/1000 | 1.13±0.04 | 30 |
| | 1/5000 | 0.73±0.05 | 20 |
| 0.1% Triton® X-100 | | 3.74±0.07 | 100 |

### Example 7. Antibody immunoassay with a solid support of polyacrylamide-enmeshed liposomes in a multiwell microtiter plate

To each well of a multiwell microtiter plate containing 10% polyacrylamide wells as shown in Figure 1C, is added a volume of an aqueous solution of 30% acrylamide-0.8% methylene-bis-acrylamide containing the liposomes of Example 2.1 with occluded calcein and trinitrophenyl-phosphatidyl-ethanolamine in the outer surface. Then, as shown in Figure 2, immediately after adding the Temed and ammonium persulfate to initiate the acrylamide polymerization , the mold A is introduced to give an ensemble as shown in C. After polymerization occurs, the mold A is removed to give a multiwell microtiter plate with the geometry shown in D. Wells are thus created which have a transparent polymerized acrylamide layer lined by an additional layer of polyacrylamide-enmeshed liposomes with a specific hapten on their surface and with self-quenched calcein within their inner volume.

To these wells, fresh immune serum containing anti-trinitrobenzene antibodies is added directly as shown in E. After incubation for 15 to 30 min, complement-mediated specific lysis of the liposomes occurs by complement present in immune sera and calcein is released (G). The black dots in (G) represent the calcein fluorescence in the polyacrylamide transparent layer, that is very high. When non-immune serum, i.e. serum not containing antibodies against the hapten present at the liposome surface, are added to the well (F), almost no lysis occurs.

### Example 8. Immunoassay of an antigen of hapten with polyacrylamide-enmeshed liposomes with occluded fluorophore in the presence of a cytolytic agent

Liposomes made of phospholipids, cholesterol and α-tocopherol, are loaded with a fluorophore. They are enmeshed in a 10% acrylamide-0.26% methylene-bis-acrylamide gel.

### 8.1 Cytolytic peptide-based immunoassay

CH-1, a melittin homolog of the formula 
NH₂-Gly-Trp-Gly-Ala-Val-Leu-Lys-Val-Leu-Thr-Thr
-Gly-Leu-Pro-Ala-Leu-Ile-Ser-Cys-Ile-Lys-Gln-amide
 with molecular weight of 2,000 daltons, is derivatized to contain an attached hapten (a peptide epitope or other) and the [CH-1-hapten] conjugate is reacted with a monoclonal antibody such that the antibody and the [CH-1-hapten] conjugate form a 1:1 complex. Since the antibody has a molecular weight of 150,000 daltons, the complex will have a molecular weight of about 152,000. At this size, the complex is not able to penetrate through the acrylamide gel matrix. The free [CH-1-hapten] conjugate, however, when released from the interaction with the monoclonal antibody, readily penetrates the gel matrix. It then associates to form a channel through the liposome membrane that releases the occluded fluorophore into the medium.

The [monoclonal antibody-CH-1-hapten] complex is incubated with serum or plasma containing the antigen to be determined, the antigen being similar in structure to the hapten attached to the CH-1. Because of this similarity in structure, the hapten displaces the [CH-1-hapten] conjugate from the monoclonal antibody. Polyacrylamide-enmeshed liposomes containing a fluorophore are washed by sedimentation, to reduce any interference by the fluorophore and then added to the sample. The [CH-1-hapten] conjugate diffuses into the acrylamide gel matrix, lyses the liposomes and releases the fluorophore, that is measured.

### 8.2 Phospholipase-based immunoassay

Phospholipase (molecular weight 12,000 daltons) is derivatized to contain an attached hapten (epitope). This [phospholipase-hapten] conjugate is mixed with a monoclonal antibody to give a 1:1 complex. Since the antibody has a molecular weight of 150,000 daltons, the complex has a molecular weight of about 162,000, and is not able to penetrate into the polyacrylamide gel matrix. However, the free [phospholipase-hapten] conjugate readily penetrates the gel matrix, when released from the interaction with the monoclonal antibody, thus lysing the liposomes to release the fluorophore.

The [monoclonal antibody-phospholipase-hapten] complex is incubated in the presence of albumin with serum or plasma containing the antigen to be determined, the antigen being similar in structure to the hapten attached to the phospholipase. Because of this similarity in structure, the hapten displaces the [phospholipase-hapten] conjugate from the monoclonal antibody. Polyacrylamide-enmeshed liposomes containing a fluorophore are washed (to remove free fluorophore) and are then added to the sample. The free [phospholipase-hapten] conjugate diffuses into the gel and lyses the liposomes releasing the fluorophore, that is measured.

## Claims

1. A liposome direct immunoassay of an antibody, antigen, or hapten in a sample which comprises:
a) addition of the sample containing the antibody, antigen or hapten to polymer-enmeshed liposomes having a marker occluded within their inner volume;
b) incubation of the suspension with an agent to cause liposome lysis with subsequent release of the marker into the supernatant;
c) sedimentation of the suspension under normal gravity; and
d) measurement of the marker released from the lysed liposomes in the supernatant whereby the amount of marker indicates the concentration of the antibody, antigen or hapten in the sample.

2. A liposome direct immunoassay according to claim 1 wherein the agent causing liposome lysis in step (b) is complement.

3. A liposome direct immunoassay of an antibody according to claim 1 or 2, wherein
in step (a) immune serum containing the antibody is added to low cross-linked polymer-enmeshed liposomes having attached to their outer surface a hapten recognized by the antibody, and
in step (b) the incubation of the suspension is carried out in the presence of complement, either present in the immune serum or externally added complement, to cause antibody-dependent complement-mediated liposome lysis.

4. A liposome direct immunoassay of an antigen or a hapten according to claim 1 or 2, wherein
in step (a) the sample containing the antigen or hapten is first neutralized with an excess of the respective antibody and it is then added to the polymer-enmeshed liposomes having attached to their outer surface a hapten recognized by the antibody, and
in step (b) the incubation of the suspension is carried out in the presence of complement, either present in the sample or externally added complement, to cause antibody-dependent complement-mediated liposome lysis.

5. A liposome direct immunoassay according to any of claims 1 to 4 wherein the liposomes are enmeshed in a polymer network consisting of a gel that does not damage the liposomes nor interferes with the assay.

6. A liposome direct immunoassay according to claim 5 wherein the gel consists of a high molecular weight material selected from cross-linked polyacrylamide, polymethacrylic acid, and polyethylene oxide.

7. A liposome direct immunoassay according to claim 6 wherein the polyacrylamide gel matrix is obtained by polymerizing about 5% acrylamide in the presence of about 0.13% of the cross-linking agent N,N'-methylene-bis-acrylamide.

8. A liposome direct immunoassay according to claim 1 wherein the agent causing liposome lysis in step (b) is a cytolytic agent and the polymer network enmeshing the liposomes acts as a selective barrier for diffusion of molecules of different molecular weight to the liposome surface.

9. A liposome direct immunoassay according to claim 8 wherein the polymer network allows the diffusion of a [cytolytic agent-hapten] conjugate, but not of an [antibody-cytolytic agent-hapten] complex.

10. A liposome direct immunoassay according to claim 8 wherein the polymer is cross-linked polyacrylamide obtained by polymerizing about 10-12% acrylamide in the presence of about 0.26-0.31% N, N'-methylene-bis-acrylamide.

11. A liposome direct immunoassay according to any of claims 8 to 10 for direct immunoassay of an antibody in a sample in the presence of a cytolytic agent, which comprises:
(a) incubation of the sample with known concentrations of a conjugate of a cytolytic agent and a hapten recognized by the antibody, in excess to the antibody to be measured, so as to form an [antibody-cytolytic agent-hapten] complex;
(b) addition of the sample containing the complex and free [cytolytic agent-hapten] conjugate to polymer-enmeshed liposomes having an occluded marker, such that only the conjugate will penetrate into and reach the liposome surface causing liposome lysis and subsequent release of the marker into the supernatant;
c) sedimentation of the suspension under normal gravity, and
d) measurement of the marker released from the lysed liposomes in the supernatant.

12. A liposome direct immunoassay according to any of claims 8 to 10 for direct immunoassay of an antigen or a hapten in a sample, which comprises:
(a) incubation of a mixture of known amounts of an antibody against the antigen or hapten with a conjugate of a cytolytic agent with a hapten recognized by the antibody, resulting in neutralization and formation of a 1:1 [antibody-cytolytic agent-hapten] complex, with no free antibody or [cytolytic agent-hapten] conjugate;
(b) addition of mixture obtained in (a) to the sample containing the antigen or hapten to be measured, so that the antigen or hapten will compete for the antibody in the complex and will release [cytolytic agent-hapten] conjugate;
(c) addition of highly crosslinked polymer-enmeshed liposomes having a marker occluded within their inner volume, to mixture (b) and incubation of the suspension, such as to cause liposome lysis by the free [cytolytic agent-hapten] conjugate, and release of the marker into the supernatant;
(d) sedimentation of the suspension; and
(e) measurement of the marker released from the lysed liposomes in the supernatant.

13. A liposome direct immunoassay according to any of claims 1 to 12 where the polyacrylamide-enmeshed liposomes are kept solid-like and form a receptacle for the sample.

14. A liposome direct immunoassay according to claim 13 which comprises:
a) addition of a 10% acrylamide-methylene-bis-acrylamide solution to the wells of a plastic multiwell microtiter plate and polymerization in the presence of a catalyst and a mold, and withdrawal of the mold such as to form a transparent polyacrylamide layer coating the well;
b) addition of a 10% acrylamide-methylene-bis-acrylamide solution and liposomes containing occluded marker to the polyacrylamide-coated wells of step (a), polymerization in the presence of a catalyst and a mold, and withdrawal of the mold such as to obtain a well of polyacrylamide-enmeshed liposomes above the transparent polyacrylamide layer;
c) addition of the sample to the wells; and
d) estimation/measurement of the marker released from the lysed liposome into the transparent polyacrylamide layer.

15. A liposome direct immunoassay kit for detecting an antibody, antigen or hapten in a sample which comprises:
(a) polymer-enmeshed liposomes having a marker occluded within their inner volume; and
(b) an agent to cause liposome lysis with subsequent release of the marker.

16. A liposome direct immunoassay kit according to claim 15, wherein said agent causing liposome lysis of (b) is complement.

## Patentansprüche

1. Direkter Liposomen-lmmuntest für einen Antikörper, ein Antigen oder Hapten in einer Probe, der umfaßt:
(a) Zugabe der einen Antikörper, ein Antigen oder ein Hapten enthaltenden Probe zu Polymer-eingebetteten Liposomen, die einen Marker in ihrem inneren Volumen eingeschlossen haben;
(b) Inkubation der Suspension mit einem Agens, um die Lyse der Liposomen mit darauffolgender Freisetzung des Markers in den Überstand zu bewirken;
(c) Sedimentation der Suspension unter normaler Schwerkraft; und
(d) Messung des aus den lysierten Liposomen in den Überstand freigesetzten Markers, wobei die Menge des Markers die Konzentration von Antikörper, Antigen oder Hapten in der Probe anzeigt.

2. Direkter Liposomen-Immuntest nach Anspruch 1, worin das die Lyse der Liposomen in Schritt (b) verursachende Agens Komplement ist.

3. Direkter Liposomen-Immuntest für einen Antikörper nach Anspruch 1 oder 2, wobei
in Schritt (a) den Antikörper enthaltendes Immunserum zu Liposomen gegeben wird, die in gering vernetztem Polymer eingebettet sind, und die an ihrer äußeren Oberfläche ein Hapten gebunden haben, das von dem Antikörper erkannt wird, und
in Schritt (b) die Inkubation der Suspension in Gegenwart von entweder im Immunserum vorhandenem oder extern zugegebenem Komplement durchgeführt wird, um eine Antikörper-abhängige, Komplement-vermittelte Liposomenlyse zu bewirken.

4. Direkter Liposomen-Immuntest für ein Antigen oder ein Hapten nach Anspruch 1 oder 2, wobei
in Schritt (a) die das Antigen oder Hapten enthaltende Probe erst mit einem Überschuß des jeweiligen Antikörpers neutralisiert wird und dann die Probe zu den Polymer-eingebetteten Liposomen gegeben wird, die an ihrer äußeren Oberfläche ein vom Antikörper erkanntes Hapten gebunden haben, und
in Schritt (b) die Inkubation der Suspension in Gegenwart von entweder in der Probe vorhandenem oder extern zugegebenem Komplement durchgeführt wird, um eine Antikörper-abhängige, Komplement-vermittelte Liposomenlyse zu bewirken.

5. Direkter Liposomen-Immuntest nach einem der Ansprüche 1 bis 4, wobei die Liposomen in ein aus einem Gel bestehenden Polymernetzwerk eingebettet sind, wobei das Gel weder die Liposomen beschädigt noch den Test stört.

6. Direkter Liposomen-Immuntest nach Anspruch 5, wobei das Gel aus Material mit hohem Molekulargewicht besteht, ausgewählt aus vernetztem Polyacrylamid, Polymethacrylsäure und Polyethylenoxid.

7. Direkter Liposomen-Immuntest nach Anspruch 6, wobei die Polyacrylamidgelmatrix durch die Polymerisation von etwa 5% Polyacrylamid in Gegenwart von etwa 0,13% des Vernetzungsmittels N,N'-Methylenbisacrylamid erhalten wird.

8. Direkter Liposomen-Immuntest nach Anspruch 1, wobei das die Liposomenlyse in Schritt (b) bewirkende Agens ein cytolytisches Agens ist und das die Liposomen einbettende Polymernetzwerk als ein selektives Hindernis für die Diffusion von Molekülen verschiedenen Molekulargewichts zu der Liposomenoberfläche wirkt.

9. Direkter Liposomen-Immuntest nach Anspruch 8, wobei das Polymernetzwerk die Diffusion eines [cytolytisches Agens-Hapten]-Konjugats aber nicht eines [Antikörper-cytolytisches Agens-Hapten]-Komplexes erlaubt.

10. Direkter Liposomen-Immuntest nach Anspruch 8, wobei das Polymer vernetztes Polyacrylamid ist, das durch die Polymerisation von etwa 10-12% Acrylamid in Gegenwart von etwa 0,26 bis etwa 0,31% N,N'-Methylenbisacrylamid erhalten wird.

11. Direkter Liposomen-Immuntest nach einem der Ansprüche 8 bis 10 für einen direkten Immuntest eines Antikörpers in einer Probe in Gegenwart eines cytolytischen Agens, welcher umfaßt:
(a) Inkubation einer Probe mit bekannten Konzentrationen eines Konjugats aus einem cytolytischen Agens und einem von dem Antikörper erkannten Hapten, das in einem Überschuß zu dem zu messenden Antikörper vorliegt, um so einen [Antikörper-cytolytisches Agens-Hapten]-Komplex zu erzeugen;
(b) Zugabe der den Komplex und freies [cytolytisches Agens-Hapten]-Konjugat enthaltenden Probe zu Polymer-eingebetteten, einen Marker einschließenden Liposomen, so daß nur das Konjugat eindringen und die Oberfläche der Liposomen erreichen wird und die Lyse der Liposomen und anschließende Freisetzung der Marker in den Überstand verursacht;
(c) Sedimentation der Suspension unter normaler Schwerkraft; und
(d) Messung des aus den lysierten Liposomen in den Überstand freigesetzten Markers.

12. Direkter Liposomen-Immuntest nach einem der Ansprüche 8 bis 10 für einen direkten Immuntest eines Antigens oder eines Haptens in einer Probe, der umfaßt:
(a) Inkubation eines Gemisches mit bekannten Mengen eines Antikörpers gegen das Antigen oder Hapten mit einem Konjugat aus einem cytolytischen Agens mit einem Hapten, das von dem Antikörper erkannt wird, die zur Neutralisation und Erzeugung eines 1:1 [Antiköper-cytolytisches Agens-Hapten]-Komplexes führt, wobei kein freier Antikörper oder [cytolytisches Agens-Hapten]-Konjugat vorliegt;
(b) Zugabe von einem aus (a) erhaltenen Gemisch zu der das zu messende Antigen oder Hapten enthaltenden Probe, so daß das Antigen oder Hapten um den Antikörper in dem Komplex konkurriert und [cytolytisches Agens-Hapten]-Konjugat freisetzt;
(c) Zugabe von Liposomen, die in stark vernetztem Polymer eingebettet sind und die einen Marker in ihrem inneren Volumen einschließen, zum Gemisch (b) und Inkubation der Suspension, so daß Liposomenlyse durch das freie [cytolytische Agens-Hapten]-Konjugat und Freisetzung des Markers in den Überstand verursacht wird;
(d) Sedimentation der Suspension; und
(e) Messung des aus den lysierten Liposomen in den Überstand freigesetzten Markers.

13. Direkter Liposomen-Immuntest nach einem der Ansprüche 1 bis 12, wobei die Polyacrylamid-eingebetteten Liposomen in einer Festkörper-ähnlichen Form gehalten werden und einen Behälter für die Probe bilden.

14. Direkter Liposomen-Immuntest nach Anspruch 13, der umfaßt:
(a) Zugabe einer 10%igen Acrylamid-Methylenbisacrylamid-Lösung zu den Vertiefungen einer viele Vertiefungen besitzenden Microtiterplatte aus Plastik und Polymerisation in Gegenwart eines Katalysators und einer Form sowie Wegnahme der Form, so daß eine transparente, die Vertiefung beschichtende Polyacrylamidschicht erzeugt wird;
(b) Zugabe einer 10%igen Acrylamid-Methylenbisacrylamid-Lösung und von einen eingeschlossenen Marker enthaltenden Liposomen zu den Polyacrylamid-beschichteten Vertiefungen des Schritts (a), Polymerisation in Gegenwart eines Katalysators und einer Form sowie Wegnahme der Form, so daß eine Vertiefung mit Polyacrylamid-eingebetteten Liposomen oberhalb der transparenten Polyacrylamidschicht erhalten wird;
(c) Zugabe der Probe zu den Vertiefungen; und
(d) Abschätzung/Messung des aus den lysierten Liposomen in die transparente Polyacrylamidschicht freigesetzten Markers.

15. Direkter Liposomen-lmmuntest-Kit zum Nachweis eines Antikörpers, Antigens oder Haptens in einer Probe, der umfaßt:
(a) Polymer-eingebettete Liposomen, die einen Marker in ihrem inneren Volumen eingeschlossen haben; und
(b) ein Agens, das Lyse der Liposomen und nachfolgende Freisetzung des Markers bewirkt.

16. Direkter Liposomen-lmmuntest-Kit nach Anspruch 15, wobei das die Liposomenlyse bewirkende Agens von (b) Komplement ist.

## Revendications

1. Essai immunologique direct à l'aide de liposomes d'un anticorps, antigène ou haptène dans un échantillon, comprenant les étapes consistant à :
a) ajouter l'échantillon contenant l'anticorps, antigène ou haptène à des liposomes piégés dans un polymère et renfermant un marqueur dans leur volume interne ;
b) incuber la suspension avec un agent afin de provoquer la lyse des liposomes, suivie de la libération du marqueur dans le surnageant ;
c) laisser la suspension se sédimenter sous l'action de la gravité normale ; et
d) mesurer le marqueur libéré par les liposomes lysés dans le surnageant, la quantité de marqueur indiquant la concentration de l'anticorps, antigène ou haptène dans l'échantillon.

2. Essai immunologique direct à l'aide de liposomes selon la revendication 1, dans lequel l'agent provoquant la lyse des liposomes à l'étape (b) est du complément.

3. Essai immunologique direct à l'aide de liposomes d'un anticorps selon la revendication 1 ou la revendication 2, dans lequel,
à l'étape (a), on ajoute du sérum immun contenant l'anticorps aux liposomes piégés dans un polymère faiblement réticulé et possédant, fixé sur leur surface extérieure, un haptène reconnu par l'anticorps ; et
à l'étape (b), on réalise l'incubation de la suspension en présence de complément, soit présent dans le sérum immun, soit du complément extérieur ajouté, afin de provoquer la lyse des liposomes en fonction de l'anticorps avec médiation par le complément.

4. Essai immunologique direct à l'aide de liposomes d'un antigène ou haptène selon la revendication 1 ou la revendication 2, dans lequel,
à l'étape (a), on neutralise d'abord l'échantillon contenant l'antigène ou haptène avec un excès de l'anticorps correspondant et on l'ajoute ensuite aux liposomes piégés dans le polymère et possédant, fixé sur leur surface extérieure, un haptène reconnu par l'anticorps ; et
à l'étape (b), on réalise l'incubation de la suspension en présence de complément, soit présent dans l'échantillon, soit du complément extérieur ajouté, afin de provoquer la lyse des liposomes en fonction de l'anticorps avec médiation par le complément.

5. Essai immunologique direct à l'aide de liposomes selon l'une quelconque des revendications 1 à 4, dans lequel les liposomes sont piégés dans un réseau de polymère consistant en un gel qui ne détériore pas les liposomes et qui ne perturbe pas l'essai.

6. Essai immunologique direct à l'aide de liposomes selon la revendication 5, dans lequel le gel se compose d'un matériau à haut poids moléculaire choisi dans le groupe comprenant le polyacrylamide réticulé, l'acide polyméthacrylique et l'oxyde de polyéthylène.

7. Essai immunologique direct à l'aide de liposomes selon la revendication 6, dans lequel la matrice de gel de polyacrylamide est obtenue en polymérisant environ 5 % d'acrylamide en présence d'environ 0,13 % de l'agent de réticulation N,N'-méthylène-bis-acrylamide.

8. Essai immunologique direct à l'aide de liposomes selon la revendication 1, dans lequel l'agent qui provoque la lyse des liposomes à l'étape (b) est un agent cytolytique et le réseau de polymère qui piège les liposomes agit comme une barrière sélective pour la diffusion des molécules de poids moléculaires différents vers la surface des liposomes.

9. Essai immunologique direct à l'aide de liposomes selon la revendication 8, dans lequel le réseau de polymère permet la diffusion d'un conjugué [agent cytolytique/haptène] mais pas celle d'un complexe [anticorps/agent cytolytique/haptène].

10. Essai immunologique direct à l'aide de liposomes selon la revendication 8, dans lequel le polymère est du polyacrylamide réticulé obtenu en polymérisant environ 10 à 12 % d'acrylamide en présence d'environ 0,26 à 0,31 % de N,N'-méthylène-bis-acrylamide.

11. Essai immunologique direct à l'aide de liposomes selon l'une quelconque des revendications 8 à 10, destiné à l'essai immunologique direct d'un anticorps dans un échantillon en présence d'un agent cytolytique, comprenant les étapes consistant à :
a) incuber l'échantillon avec des concentrations connues d'un conjugué d'un agent cytolytique et d'un haptène reconnu par l'anticorps, en excès par rapport à l'anticorps à mesurer, de manière à former un complexe [anticorps/agent cytolytique/haptène] ;
b) ajouter l'échantillon contenant le complexe et du conjugué [agent cytolytique/haptène] libre aux liposomes piégés dans le polymère et renfermant un marqueur, de telle sorte que seul le conjugué pénètre dans et atteigne la surface des liposomes, provoquant la lyse des liposomes suivie de la libération du marqueur dans le surnageant ;
c) laisser la suspension se sédimenter sous l'action de la gravité normale ; et
d) mesurer le marqueur libéré par les liposomes lysés dans le surnageant.

12. Essai immunologique direct à l'aide de liposomes selon l'une quelconque des revendications 8 à 10, destiné à l'essai immunologique direct d'un antigène ou d'un haptène dans un échantillon, comprenant les étapes consistant à :
a) incuber un mélange de quantités connues d'un anticorps dirigé contre l'antigène ou haptène avec un conjugué d'un agent cytolytique et d'un haptène reconnu par l'anticorps, avec pour résultat la neutralisation et la formation d'un complexe [anticorps/agent cytolytique/haptène] 1:1 sans anticorps libre ni conjugué [agent cytolytique/haptène] ;
b) ajouter le mélange obtenu en (a) à l'échantillon contenant l'antigène ou haptène à mesurer de telle sorte que l'antigène ou haptène va entrer en compétition pour l'anticorps dans le complexe et libérer du conjugué [agent cytolytique/haptène] ;
c) ajouter au mélange (b) des liposomes piégés dans un polymère hautement réticulé et renfermant un marqueur dans leur volume interne et incuber la suspension de manière à provoquer la lyse des liposomes par le conjugué [agent cytolytique/haptène] libre et la libération du marqueur dans le surnageant ;
d) laisser la suspension se sédimenter ; et
e) mesurer le marqueur libéré par les liposomes lysés dans le surnageant.

13. Essai immunologique direct à l'aide de liposomes selon l'une quelconque des revendications 1 à 12, dans lequel les liposomes piégés dans du polyacrylamide sont conservés sous forme solide et forment un réceptacle pour l'échantillon.

14. Essai immunologique direct à l'aide de liposomes selon la revendication 13, comprenant les étapes consistant à :
a) ajouter une solution à 10 % d'acrylamide-méthylène-bis-acrylamide dans les cupules d'une microplaque à cupules multiples en plastique et polymériser en présence d'un catalyseur et d'un moule, puis à retirer le moule de manière à former une couche de polyacrylamide transparente qui sensibilise la cupule ;
b) ajouter une solution à 10 % d'acrylamide-méthylène-bis-acrylamide et des liposomes renfermant un marqueur aux cupules sensibilisées par le polyacrylamide de l'étape (a) et polymériser en présence d'un catalyseur et d'un moule, puis à retirer le moule de manière à former une cupule de liposomes piégés dans un polyacrylamide pardessus la couche de polyacrylamide transparente ;
c) ajouter l'échantillon dans les cupules ; et
d) estimer/mesurer le marqueur libéré par les liposomes lysés dans la couche de polyacrylamide transparente.

15. Nécessaire d'essai immunologique direct à l'aide de liposomes pour la détection d'un anticorps, antigène ou haptène dans un échantillon, comprenant :
a) des liposomes piégés dans un polymère renfermant un marqueur dans leur volume interne ; et
b) un agent servant à provoquer la lyse des liposomes. suivie de la libération du marqueur.

16. Nécessaire d'essai immunologique direct à l'aide de liposomes selon la revendication 15, dans lequel ledit agent provoquant la lyse des liposomes de l'étape (b) est du complément.
